(19) Europäisches Patentamt
European Patent Office
Office européen des brevets

(11) EP 1 525 198 B1

(12) **FASCICULE DE BREVET EUROPEEN**

(45) Date de publication et mention de la délivrance du brevet:
**07.12.2005 Bulletin 2005/49**

(21) Numéro de dépôt: **03753652.1**

(22) Date de dépôt: **17.07.2003**

(51) Int Cl.[7]: **C07D 403/04**, A61K 31/404, A61P 5/00

(86) Numéro de dépôt international:
**PCT/FR2003/002262**

(87) Numéro de publication internationale:
**WO 2004/009585 (29.01.2004 Gazette 2004/05)**

# (54) DERIVES D'ACYLOXYPYRROLIDINE ET LEUR UTILISATION EN TANT QUE LIGANDS DES RECEPTEURS V1B OU V1B ET V1A DE AVP

ACYLOXYPYRROLIDINDERIVATE UND DEREN VERWENDUNG ALS LIGANDEN DER V1B ODER V1B UND V1A REZEPTOREN VON AVP

ACYLOXYPYRROLIDINE DERIVATIVES, PREPARATION AND THERAPEUTIC USE THEREOF

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **19.07.2002 FR 0209242**

(43) Date de publication de la demande:
**27.04.2005 Bulletin 2005/17**

(73) Titulaire: **Sanofi-Aventis**
**75013 Paris (FR)**

(72) Inventeurs:
- **AULOMBARD, Alain**
  **F-34000 Montpellier (FR)**
- **GARCIA, Georges**
  **F-34110 Frontignan (FR)**
- **PRADINES, Antoine**
  **F-31120 Roquettes (FR)**
- **SERRADEIL-LE GAL, Claudine**
  **F-31750 Escalquens (FR)**
- **WAGNON, Jean**
  **F-34070 Montpellier (FR)**

(74) Mandataire: **Tsitini-Souleau, Maria et al**
**Sanofi-Aventis**
**174 avenue de France**
**75013 Paris (FR)**

(56) Documents cités:
**WO-A-01/55130**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des dérivés d'acyloxypyrrolidine, leur préparation et leur application en thérapeutique.

Les composés selon la présente invention présentent une affinité et une sélectivité pour les récepteurs $V_{1b}$ ou à la fois pour les récepteurs $V_{1b}$ et $V_{1a}$ de l'arginine-vasopressine (AVP).

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés, en particulier chez le rat et l'homme et ils régulent la majorité des effets connus de l'AVP : l'agrégation plaquettaire ; les contractions utérines ; la contraction des vaisseaux ; la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire,...).

Les récepteurs $V_{1b}$ ont été initialement identifiés dans l'adénohypophyse de différentes espèces animales (rat, porc, boeuf, mouton...) y compris chez l'homme (S. JARD et al., Mol. Pharmacol., 1986, 30, 171-177 ; Y. ARSENIJEVIC et al., J. Endocrinol., 1994, 141, 383-391 ; J. SCHWARTZ et al., Endocrinology, 1991, 129 (2), 1107-1109 ; Y. DE KEYSER et al., FEBS Letters, 1994, 356, 215-220) où ils stimulent la libération d'hormone adrénocorticotrophique par l'AVP et potentialisent les effets du CRF sur la libération d'ACTH (G.E. GILLIES et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. DE KEYSER, FEBS Letters, 1994, 356, 215-220 ; T. SUGIMOTO et al., J. Biol. Chem., 1994, 269 (43), 27088-27092 ; M. SAITO et al., Biochem. Biophys. Res. Commun., 1995, 212 (3), 751-757 ; S.J. LOLAIT et al., Neurobiology, 1996, 92, 6783-6787 ; M.A. VENTURA et al., Journal of Molecular Endocrinology, 1999, 22, 251-260) et différentes études (hybridation *in situ,* PCR, de l'anglais Polymerase Chain Reaction, ...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régule le pancréas endocrine en stimulant la sécrétion d'insuline et de glucagon (B. LEE et al., Am. J. Physiol. 269 (Endocrinol. Metab. 32) : E1095-E1100, 1995) ou la production de catécholamines dans la medullo-surrénale qui est le siège d'une synthèse locale d'AVP (E. GRAZZINI et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétants de l'AVP et induisant de ce fait une production soutenue de catécholamines à l'origine d'hypertensions résistantes aux antagonistes des récepteurs de l'angiotensine II et aux inhibiteurs de l'enzyme de conversion. Le cortex surrénalien est aussi riche en récepteurs $V_{1a}$ impliqués dans la production de gluco- et minéralo-corticoïdes (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1b}$ et/ou $V_{1a}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1b}$ sont aussi considérés comme des marqueurs de tumeurs. Par exemple, les tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroïdiens, induisant un syndrome de Cushing dans certains cas (J. BERTHERAT et al., Eur. J. Endocrinol., 1996, 135,173 ; G.A. WITTERT et al., Lancet, 1990, 335, 991-994 ; G. DICKSTEIN et al., J. Clin. Endocrinol. Metab., 1996, 81 (8), 2934-2941) surexpriment les récepteurs $V_{1b}$. Les récepteurs $V_{1a}$ sont, quant à eux, des marqueurs plus spécifiques des cancers pulmonaires à petites cellules (SCLC) (P.J. WOLL et al., Biochem. Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. SUGIMOTO et al., Molecular cloning and functional expression of $V_{1b}$ receptor gene, dans Neurohypophysis : Récent Progress of Vasopressin and Oxytocin Research ; T. SAITO, K. KUROKAWA and S. YOSHIDA ed., Elvesier Science, 1995, 409-413).

La demande de brevet internationale WO 01/55130 décrit une famille de composés qui présentent une affinité et une sélectivité pour les récepteurs $V_{1b}$ ou à la fois pour les récepteurs $V_{1b}$ et $V_{1a}$ de l'arginine-vasopressine.

Plus particulièrement, un antagoniste sélectif des récepteurs $V_{1b}$, le (2S,4R)-1-[5-chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre, (ci-après dénommé composé A) a été décrit (WO 01/55130 ; J. Pharmacol. Exp. Ther., 2002, 300 (3), 1122-1130).

On a maintenant trouvé de nouveaux composés, dérivés d'acyloxypyrrolidine qui présentent une affinité et une sélectivité pour les récepteurs $V_{1b}$ ou à la fois pour les récepteurs $V_{1b}$ et $V_{1a}$ de l'arginine-vasopressine.

La présente invention a pour objet des composés répondant à la formule (I) :

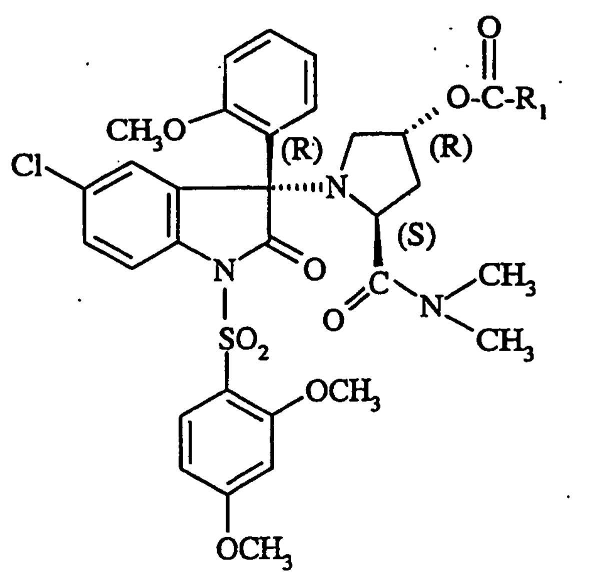

(I)

dans laquelle :

- $R_1$ représente un atome d'hydrogène, un $(C_1-C_6)$alkyle, un $(C_3-C_6)$cycloalkyle, un groupe $-CH_2CH_2COOH$ ou un groupe $-NR_2R_3$ ;
- $R_2$ et $R_3$ représentent chacun indépendamment un atome d'hydrogène ou un $(C_1-C_6)$alkyle.

Les composés de formule (I) comprennent trois atomes de carbone asymétriques, l'atome de carbone portant le substituant $-CON(CH_3)_2$ a la configuration (S), l'atome de carbone portant le substituant $-OCOR_1$ a la configuration (R), et l'atome de carbone en position 3 de l'indole-2-one a la configuration (R).

Les composés de formule (I) peuvent exister à l'état de bases ou de sels d'addition à des bases organiques ou inorganiques tels que les sels avec des métaux alcalins ou alcalino-terreux, ou les sels avec des amines organiques ou inorganiques. De tels sels d'addition font partie de l'invention.

Ces sels sont avantageusement préparés avec des bases pharmaceutiquement acceptables, mais les sels d'autres bases utiles, par exemple, pour la purification ou l'isolement des composés de formule (I) font également partie de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats

font également partie de l'invention.

Par alkyle on entend un radical alkyle linéaire ou ramifié de un à six atomes de carbone tel que le radical méthyle, éthyle, propyle, isopropyle, butyle, isobutyle, *tert*-butyle, pentyle, isopentyle, hexyle, isohexyle.

Par cycloalkyle on entend un radical alkyle cyclique de trois à six atomes de carbone tel que le radical cyclopropyle, cyclobutyle, cyclopentyle, cyclohexyle.

Parmi les composés de formule (I), objets de l'invention, on peut citer les composés préférés qui se définissent comme suit :

- $R_1$ représente un atome d'hydrogène, un radical méthyle, un radical éthyle, un radical isopropyle, un radical cyclohexyle, un groupe $-CH_2CH_2COOH$, un groupe amino ou un groupe diméthylamino ;

à l'état de base ou de sel d'addition à une base organique ou inorganique, ainsi qu'à l'état d'hydrate ou de solvat.

Parmi les composés de formule (I) objets de l'invention, on peut notamment citer les composés suivants :

- Acétate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle ;
- Propionate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle ;
- Formiate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle ;
- Cyclohexanecarboxylate de (3R,5S)-1-[(3R)-5-chloro-1-[(2,4-diméthoxyphényl) sulfonyl]-3-(2-méthoxyphényl)-2-oxo-2,3-dihydro-1*H*-indol-3-yl]-5-[(diméthylamino)carbonyl]-3-pyrrolidinyle ;
- 2-Méthylpropanoate de (3R,5S)-1-[(3R)-5-chloro-1-[(2,4-diméthoxyphényl) sulfonyl]-3-(2-méthoxyphényl)-2-oxo-2,3-dihydro-1*H*-indol-3-yl]-5-[(diméthylamino)carbonyl]-3-pyrrolidinyle;
- Acide 4-[[(3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyl]oxy]-4-oxobutanoïque ;
- (2S,4R)-4-[(aminocarbonyl)oxy]-1-[(3R)-5-chloro-1-[(2,4-diméthoxyphényl) sulfonyl]-3-(2-méthoxyphényl)-2-oxo-2,3-dihydro-1*H*-indol-3-yl]-N,N-diméthyl-2-pyrrolidinecarboxamide ;
- (2S,4R)-4-[[(diméthylamino)carbonyl]oxy]-1-[(3R)-5-chloro-1-[(2,4-diméthoxyphényl)sulfonyl]-3-(2-méthoxyphényl)-2-oxo-2,3-dihydro-1*H*-indol-3-yl]-N,N-diméthyl-2-pyrrolidinecarboxamide ;

à l'état de base ou de sel d'addition à une base organique ou inorganique, ainsi qu'à l'état d'hydrate ou de solvat.

Selon un autre de ses aspects, la présente invention a pour objet un procédé de préparation des composés de formule (I) caractérisé en ce que :

on fait réagir le (2S,4R)-1-[5-chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre, de formule :

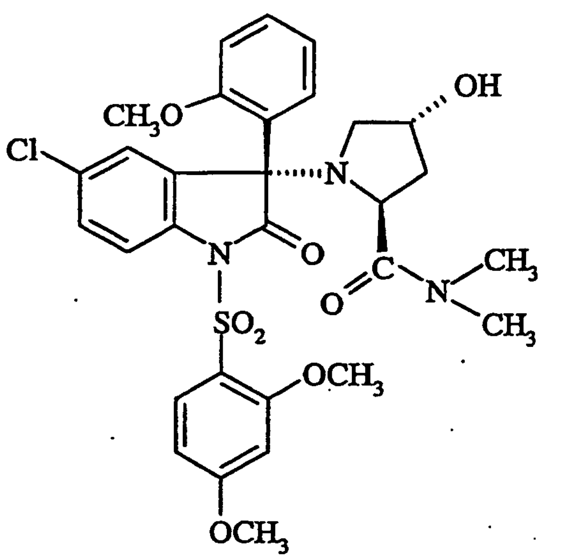

(composé A)

avec un dérivé fonctionnel d'un acide de formule :

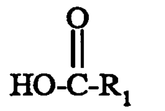 (II)

dans laquelle $R_1$ est tel que défini pour un composé de formule (I).

Eventuellement, on transforme le composé de formule (I) en l'un de ses sels d'addition à une base.

Comme dérivé fonctionnel de l'acide de formule (II) on peut utiliser le chlorure d'acide, un anhydride ou l'acide libre lui-même.

Lorsqu'on utilise un chlorure d'acide, la réaction s'effectue dans un solvant tel qu'un solvant chloré comme le dichlorométhane, le dichloroéthane, le chloroforme, un éther comme le tétrahydrofurane, le dioxane, un amide comme le N,N-diméthylformamide, en présence d'une base telle que la triéthylamine, la N-méthylmorpholine, la pyridine, la 4-diméthylaminopyridine ou la N,N-diisopropyléthylamine et à une température comprise entre -60°C et la température ambiante.

Lorsqu'on utilise un anhydride, la réaction s'effectue en l'absence ou en présence d'une base telle que la pyridine, la 4-(diméthylamino)pyridine, dans un solvant ou en l'absence de solvant à une température comprise entre la température ambiante et la température de reflux du milieu réactionnel. Lorsqu'on utilise un solvant, celui-ci peut être choisi parmi un solvant chloré comme le dichlorométhane, un solvant aromatique comme le toluène.

Un composé de formule (I) dans laquelle $R_1$ représente un groupe $-CH_2CH_2COOH$ peut également être préparé par réaction du composé A avec l'anhydride succinique, en présence d'une base telle que la pyridine, dans un solvant ou en l'absence de solvant et à une température comprise entre la température ambiante et la température de reflux du milieu réactionnel.

Lorsqu'on utilise l'acide lui-même, la réaction s'effectue en utilisant un agent de condensation tel qu'un carbodiimide comme le 1,3-dicyclohexylcarbodiimide ou le 1,3-diisopropylcarbodiimide, un imidazole comme le 1,1'-oxalyldiimidazole ou le N,N'-carbonyldiimidazole. La réaction s'effectue en l'absence ou en présence d'une base telle que la triéthylamine, la N,N-diisopropyléthylamine, la pyridine, la 4-diméthylaminopyridine ou la N-méthylmorpholine, dans un solvant tel qu'un solvant chloré comme le dichlorométhane, le dichloroéthane, le chloroforme, un ester comme l'acétate d'éthyle, un éther comme l'éther diéthylique, l'éther diisopropylique, le tétrahydrofurane, le dioxane, un nitrile comme l'acétonitrile, un amide comme le N,N-diméthylformamide, un aromatique comme le toluène, le xylène et à une température comprise entre -20°C et 80°C.

Lorsqu'on utilise l'acide lui-même, on peut également effectuer la réaction en présence d'un catalyseur acide tel qu'un acide inorganique comme l'acide chlorhydrique, l'acide bromhydrique, l'acide sulfurique, un acide organique comme l'acide acétique, l'acide formique, l'acide oxalique, l'acide p-toluènesulfonique, un acide de Lewis comme le trichlorure de bore, le trifluorure de bore ou le tribromure de bore. La réaction s'effectue dans un solvant tel qu'un solvant chloré comme le dichlorométhane, le dichloroéthane, le chloroforme, un éther comme l'éther diéthylique, l'éther diisopropylique, le tétrahydrofurane, le dioxane, une cétone comme l'acétone, la méthyléthylacétone, la méthylisobutylcétone, un nitrile comme l'acétonitrile, un amide comme le N,N-diméthylformamide et à une température comprise entre 0°C et la température de reflux du solvant.

Un composé de formule (I) dans laquelle $R_1$ représente un atome d'hydrogène peut également être préparé par réaction du composé A avec l'acide formique, en présence d'anhydride acétique et d'une base telle que la pyridine, à une température comprise entre 0°C et la température ambiante.

Selon une variante du procédé et lorsque $R_1$ représente un groupe $-NR_2R_3$ :

a) on fait réagir le composé A, en présence d'une base, avec le chloroformiate de phényle pour obtenir le composé B de formule :

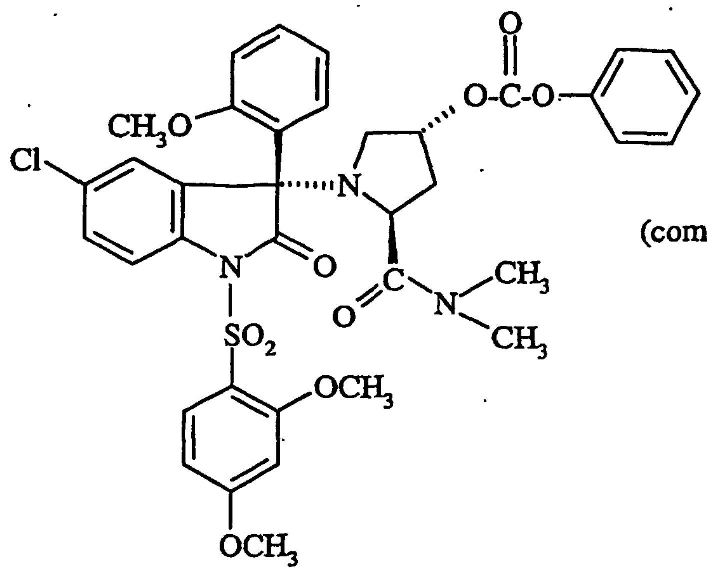

(composé B)

b) on fait réagir le composé B avec un composé de formule :

$$HNR_2R_3 \quad (III)$$

dans laquelle $R_2$ et $R_3$ sont tels que définis pour un composé de formule (I) pour obtenir un composé de formule (I) dans laquelle $R_1 = NR_2R_3$.

A l'étape a), on fait réagir le composé A avec du chloroformiate de phényle, en présence d'une base telle que la pyridine ou la triéthylamine, dans un solvant tel que le dichlorométhane ou sans solvant et à une température comprise entre 0°C et 100°C.

A l'étape b), la réaction du composé B avec le composé de formule (III) s'effectue dans un solvant tel que le dichlorométhane ou le tétrahydrofurane ou un mélange de ces solvants et à une température comprise entre -60°C et la température de reflux du solvant.

Les composés de formule (I) ainsi obtenus peuvent être ultérieurement séparés du milieu réactionnel et purifiés selon les méthodes classiques, par exemple, par cristallisation ou chromatographie.

Le composé A se prépare selon la méthode décrite dans la demande WO 01/55130.

Les dérivés fonctionnels des acides de formule (II) sont commerciaux, connus ou préparés selon des méthodes connues.

L'invention selon un autre de ses aspects, a également pour objet le composé B. Ce composé est utile comme intermédiaire de synthèse des composés de formule (I) dans laquelle $R_1 = NR_2R_3$.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les Exemples on utilise les abréviations suivantes :

AcOEt : acétate d'éthyle
Ether : éther diéthylique
Ether iso : éther diisopropylique
DCM : dichlorométhane
F : point de fusion
TA : température ambiante
Eb : température d'ébullition
CLHP : chromatographie liquide haute performance.

Les spectres de résonance magnétique du proton (RMN $^1$H) sont enregistrés à 200 MHz dans du DMSO-$d_6$,

en utilisant le pic du DMSO-$d_6$ comme référence. Les déplacements chimiques δ sont exprimés en parties par million (ppm). Les signaux observés sont exprimés ainsi : s: singulet ; se : singulet élargi ; d : doublet ; d.d : doublet dédoublé ; t : triplet ; q : quadruplet ; m : massif ; mt : multiplet.

Les spectres RMN confirment les structures des composés.

EXEMPLE 1 : composé N° 1

Acétate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle.

(I) : $R_1 = -CH_3$.

On chauffe à reflux pendant deux heures 30 minutes un mélange de 30 g de (2S,4R)-1-[5-chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre (composé A), 1,45 g de 4-(diméthylamino)pyridine et 300 ml d'anhydride acétique. Après refroidissement du mélange réactionnel à température ambiante, on ajoute 170 ml d'éthanol absolu. On concentre sous vide, extrait le résidu avec 1000 ml d'AcOEt, lave la phase organique par 670 ml d'une solution aqueuse saturée de $NH_4Cl$, deux fois par une solution aqueuse de $NaHCO_3$ et évapore le solvant sous vide. On reprend le résidu dans 190 ml de propan-2-ol, chauffe à reflux le milieu réactionnel puis refroidit à température ambiante. On concentre sous vide, reprend le résidu dans l'éther iso et laisse en cristallisation. On essore le produit cristallisé formé, le lave à l'éther iso et le sèche. On obtient 30,28 g du produit attendu, F =194-195°C.

$\alpha_D^{25}$ = -133,9° (c = 0,5 ; acétonitrile).

RMN $^1H$ : DMSO-$d_6$ : δ (ppm) : 1,7 à 2,8 : m : 13H ; 3,3 : se : 3H ; 3,7 : s : 3H ; 3,9 : s : 3H ; 4,6 : mt : 1H ; 5,2 : mt : 1H ; 6,6 à 8,2 : m : 10H.

EXEMPLE 2 : composé N° 2

Propionate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle.

(I) : $R_1 = -CH_2CH_3$.

On chauffe à reflux pendant deux heures 30 minutes un mélange de 5 g du compose A, 0,24 ml de 4-(diméthylamino)pyridine et 50 ml d'anhydride propionique. Après refroidissement du mélange réactionnel à TA, on ajoute 28 ml d'éthanol absolu. On concentre sous vide, extrait le résidu avec 160 ml d'AcOEt, lave la phase organique par 100 ml d'une solution aqueuse saturée de NaCl, trois fois par 110 ml d'une solution aqueuse à 10 % de $NaHCO_3$ et évapore le solvant sous vide. On reprend le résidu dans l'éther iso et essore le précipité formé. On obtient 4,31 g du produit attendu.

$\alpha_D^{25}$ = -107° (c = 0,5 ; acétonitrile).

RMN $^1H$ : DMSO-$d_6$ : δ (ppm) : 0,95 : t : 2H ; 1,6 à 2,7 : m : 12H ; 3,3 : se : 3H; 3,6 : s : 3H ; 3,8 : s : 3H ; 4,5 : mt : 1H ; 5,15 : mt : 1H ; 6,6 à 8.2 : m : 11H.

EXEMPLE 3 : composé N°3.

Formiate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle.

(I) : $R_1$=H.

On refroidit à 0°C 4 ml d'acide formique, ajoute, goutte à goutte, 1,6 ml d'anhydride acétique puis laisse 4 heures sous agitation à une température inférieure à 20°C. On refroidit le mélange réactionnel au bain de glace, ajoute, en 5 minutes, une solution de 0,63 g du composé A dans 7 ml de pyridine sèche préalablement refroidie au bain de glace et laisse 48 heures sous agitation à TA. On ajoute de l'eau au mélange réactionnel, extrait à l'AcOEt, lave la phase organique à l'eau, par une solution saturée de NaCl, sèche sur $Na_2SO_4$ et évapore le solvant sous vide. On chromatographie le résidu sur gel de silice en éluant par le mélange DCM/AcOEt (70/30 ; v/v). On obtient 0,27 g du produit attendu. Après concrétisation dans le mélange DCM/éther iso (80/20 ; v/v), on obtient le produit attendu sous forme d'une poudre contenant 0,5 mole d'éther iso, F = 127°C.

$\alpha_D^{25}$ = -173° (c = 0,11 ; chloroforme).

EXEMPLE 4 : composé N° 4

Cyclohexanecarboxylate de (3R,5S)-1-[(3R)-5-chloro-1-[(2,4-diméthoxyphényl) sulfonyl]-3-(2-méthoxyphényl)-2-oxo-2,3-'dihydro-1*H*-indol-3-yl]-5-[(diméthylamino)carbonyl]-3-pyrrolidinyle.

(I) : $R_1$ = 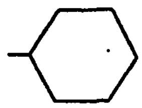

On laisse 8 jours sous agitation à TA un mélange de 1,5 g du composé A, 0,630 ml de chlorure de cyclohexanecarbonyle, 0,62 g de N,N-diisopropyléthylamine et quelques cristaux de 4-(diméthylamino)pyridine dans 20 ml de DCM. On concentre sous vide, reprend le résidu dans un mélange AcOEt/eau, alcalinise par ajout de $NaHCO_3$ solide, décante, lave deux fois la phase organique par une solution saturée de $K_2CO_3$, par une solution saturée de NaCl, sèche sur $Na_2SO_4$ et évapore le solvant sous vide. On chromatographie le résidu sur gel de silice en éluant par le mélange AcOEt/DCM (80/20 ; v/v). On reprend le produit obtenu dans 5 ml d'éther iso, laisse 48 heures sous agitation à TA, essore le précipité formé et obtient 1,0 g du produit attendu, F = 197-198°C. On concentre sous vide les jus d'essorage et cristallise le solide obtenu dans un mélange DCM/éther iso. On obtient 0,9 g de plus du produit attendu sous forme de cristaux, F = 197-200°C.

$\alpha_D^{25}$ = -144° (c = 0,18 ; chloroforme).

EXEMPLE 5 : composé N° 5

2-Méthylpropanoate de (3R,5S)-1-[(3R)-5-chloro-1-[(2,4-diméthoxyphényl) sulfonyl]-3-(2-méthoxyphényl)-2-oxo-2,3-dihydro-1*H*-indol-3-yl]-5-[(diméthylamino)carbonyl]-3-pyrrolidinyle.

$$(I) : \quad R_1 = -CH(CH_3)_2.$$

On laisse 36 heures sous agitation à TA un mélange de 1,5 g du compose A, 0,75 ml de chlorure d'isobutyryle et 1,22 g de N,N-diisopropyléthylamine dans 20 ml de DCM. On concentre sous vide, reprend le résidu à l'eau, alcalinise par ajout de $NaHCO_3$ solide, extrait à l'AcOEt, lave la phase organique à l'eau, sèche sur $Na_2SO_4$ et évapore le solvant sous vide. On chromatographie le résidu sur gel de silice en éluant par le mélange DCM/AcOEt (70/30 ; v/v). On obtient 1,17 g du produit attendu après concrétisation dans le mélange AcOEt/éther iso, F = 183-185°C.

$\alpha_D^{25}$ = -172° (c = 0,15 ; chloroforme).

EXEMPLE 6 : composé N° 6

Acide 4-[[(3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyl]oxy]-4-oxobutanoïque.

(I) : $R_1 = -CH_2CH_2COOH$.

On chauffe à 50°C pendant 5 minutes, jusqu'à dissolution, un mélange de 0,2 g du composé A, 0,2 g d'anhydride succinique et 3 ml de pyridine, puis laisse 20 heures sous agitation à 25°C. On concentre sous vide, reprend le résidu par une solution d'HCl 2N, extrait à l'éther, décante, essore le précipité formé dans la phase organique et le lave à l'éther. On obtient 0,2 g du produit attendu sous forme de cristaux, F = 225-228°C.

$\alpha_D^{25}$ = -252° (c = 0,25 ; chloroforme).

RMN $^1H$ : DMSO-$d_6$ : δ (ppm) : 1,6 à 1,9 : m : 2H ; 2,2 à 2,6 : m : 12H ; 3,0 à 3,4 : se : 3H ; 3,5 à 3,7 : se : 3H ; 3,7 à 3,9 : se : 3H ; 4,4 à 4,6 : m : 1H ; 5,1 à 5,3 : m : 1H ; 6,6 à 7,0 : m : 5H ; 7,26 : t : 1H ; 7,39 : dd : 1H ; 7,6 à 7,8 : m : 2H ; 7,94 : d : 1H ; 12,0 : se : 1H.

EXEMPLE 7 : composé N° 7

(2S,4R)-4-[(aminocarbonyl)oxy]-1-[(3R)-5-chloro-1-[(2,4-diméthoxyphényl) sulfonyl]-3-(2-méthoxyphényl)-2-oxo-2,3-dihydro-1*H*-indol-3-yl]-N,N-diméthyl-2-pyrrolidinecarboxamide.

(I) : $= R_1 = -NH_2$.

A) (2S,4R)-4-[(phénoxycarbonyl)oxy]-1-[(3R)-5-chloro-1-[(2,4-diméthoxyphényl) sulfonyl]-3-(2-méthoxyphényl)-2-oxo-2,3-dihydro-1*H*-indol-3-yl]-N,N-diméthyl-2-pyrrolidinecarboxamide.

A une solution de 1,6 g du composé A dans 20 ml de pyridine, on ajoute, à 25°C, 4 ml de chloroformiate de phényle et laisse 20 heures sous agitation à 25°C. On concentre sous vide le mélange réactionnel, reprend le résidu par une solution d'HCl 1N, extrait à l'AcOEt, sèche la phase organique sur $Na_2SO_4$ et évapore le solvant sous vide. On obtient 1,23 g du produit attendu après trituration dans l'éther iso, F = 115-125°C.

B) (2S,4R)-4-[(aminocarbonyl)oxy]-1-[(3R)-5-chloro-1-[(2,4-diméthoxyphényl) sulfonyl]-3-(2-méthoxyphényl)-2-oxo-2,3-dihydro-1*H*-indol-3-yl]-N,N-diméthyl-2-pyrrolidinecarboxamide.

On refroidit à -60°C une solution de 0,7 g du composé obtenu à l'étape précédente dans 15 ml de THF, ajoute, par barbotage, 4 g de $NH_3$ gaz et laisse 5 heures sous agitation en laissant remonter et en maintenant la température à 0°C. On concentre sous vide le mélange réactionnel et chromatographie le résidu sur gel de silice en éluant par le DCM puis à l'AcOEt. On obtient 0,37 g du produit attendu après trituration dans l'éther iso, F = 155-165°C.

$\alpha_D^{25}$ = -184° (c = 0,25 ; chloroforme).

EXEMPLE 8 : composé N° 8

(2S,4R)-4-[[(diméthylamino)carbonyl]oxy]-1-[(3R)-5-chloro-1-[(2,4-diméthoxyphényl)sulfonyl]-3-(2-méthoxyphényl)-2-oxo-2,3-dihydro-1*H*-indol-3-yl]-N,N-diméthyl-2-pyrrolidinecarboxamide.

(I) : $R_1 = -N(CH_3)_2$.

On laisse 20 heures sous agitation à 25°C un mélange de 0,35 g du composé obtenu à l'étape A de l'Exemple 7 et 10 ml d'une solution 2M de diméthylamine dans le THF. On concentre sous vide le mélange réactionnel, triture le

résidu dans l'éther iso à chaud et essore le précipité formé. On obtient 0,18 g du produit attendu, F = 138-140°C.
$\alpha_D^{25}$ = -152° (c = 0,25 ; chloroforme).

Le tableau I qui suit illustre les structures chimiques et les propriétés physiques de quelques exemples de composés selon l'invention.

TABLEAU I

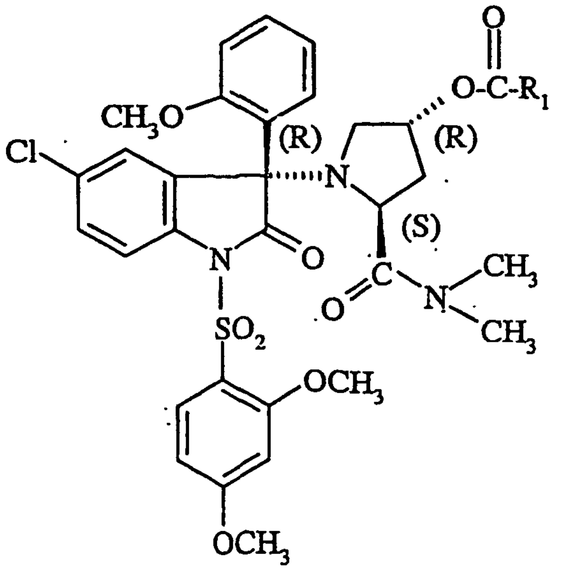

(I)

| Composés N° | $R_1$ | F°C<br>Solvant de cristallisation<br>$\alpha_D^{25}$ =<br>(chloroforme) |
|---|---|---|
| 1 | $-CH_3$ | 194-195<br>éther iso<br>-133,9° (c = 0,5 ; acétonitrile) |
| 2 | $-CH_2CH_3$ | -<br>éther iso<br>-107° (c = 0,5 ; acétonitrile) |
| 3 | H | 127<br>DCM/éther iso<br>-173° (c = 0,11) |
| 4 | 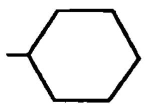 | 197-200<br>DCM/éther iso<br>-144° (c = 0,18) |
| 5 | $-CH(CH_3)_2$ | 183-185<br>AcOEt/éther iso<br>-172° (c = 0,15) |
| 6 | $-CH_2CH_2COOH$ | 225-228<br>éther<br>-252° (c = 0,25) |
| 7 | $-NH_2$ | 155-165<br>éther iso<br>-184° (c = 0,25) |
| 8 | $-N(CH_3)_2$ | 138-140<br>éther iso<br>-152° (c = 0,25) |

Les composés selon l'invention ont fait l'objet d'études biochimiques.

L'affinité des composés de formule (I) selon l'invention pour les récepteurs $V_{1b}$ de l'arginine-vasopressine a été déterminée *in vitro* en utilisant la méthode décrite par Y. DE KEYSER et al., Febs Letters, 1994, 356, 215-220. Cette méthode consiste à étudier *in vitro* le déplacement de l'arginine-vasopressine tritiée ([$^3$H]-AVP) aux récepteurs $V_{1b}$ présents sur des préparations membranaires adénohypophysaires ou cellulaires portant les récepteurs $V_{1b}$ de rat ou humains. Les concentrations inhibitrices de 50 % ($CI_{50}$) de la fixation de l'arginine-vasopressine tritiée des composés selon l'invention sont généralement inférieures à $5,0.10^{-9}$M. Par exemple, le composé de l'Exemple 1 a une $CI_{50}$ de

$3,4.10^{-9}$M pour les récepteurs $V_{1b}$ humains.

L'affinité des composés de formule (I) selon l'invention pour les récepteurs $V_{1a}$ de l'arginine-vasopressine a été déterminée *in vitro* en utilisant la méthode décrite par M. THIBONNIER et al., J. Biol. Chem., 1994, 269, 3304-3310. Cette méthode consiste à étudier *in vitro* le déplacement de l'arginine-vasopressine tritiée ($[^3H]$-AVP) aux récepteurs $V_{1a}$ présents sur des préparations membranaires ou cellulaires portant les récepteurs $V_{1a}$ de rat ou humains. Certains composés de formule (I) présentent aussi une affinité pour les récepteurs $V_{1a}$ de l'arginine-vasopressine avec des $CI_{50}$ de l'ordre de $10^{-8}$M. Par exemple, le composé de l'Exemple 1 a une $CI_{50}$ de $8,4.10^{-8}$M pour les récepteurs $V_{1a}$ humains.

Le composé A de l'art antérieur à une $CI_{50}$ de $1,0.10^{-8}$M pour les récepteurs $V_{1b}$ humains et une $CI_{50}$ de $3,1.10^{-7}$M pour les récepteurs $V_{1a}$ humains.

L'affinité des composés de formule (I) selon l'invention pour les récepteurs $V_2$ de la vasopressine a également été étudiée (méthode décrite par M. Birnbaumer et al., Nature (Lond.), 1992, 357, 333-335). Les composés étudiés sont peu ou pas affins pour les récepteurs $V_2$.

Les composés selon l'invention peuvent donc être utilisés pour la préparation de médicaments, en particulier de médicaments destinés à prévenir ou à traiter toute pathologie où l'arginine-vasopressine et/ou ses récepteurs $V_{1b}$ ou à la fois ses récepteurs $V_{1b}$ et ses récepteurs $V_{1a}$ sont impliqués.

Ainsi, selon un autre de ses aspects, l'invention a pour objet des médicaments qui comprennent un composé de formule (I), ou un sel d'addition de ce dernier à une base pharmaceutiquement acceptable, ou encore un solvat ou un hydrate du composé de formule (I).

Ainsi les composés selon l'invention peuvent être utilisés, chez l'homme ou chez l'animal, dans le traitement ou la prévention de différentes affections vasopressine-dépendantes tels que les affections cardiovasculaires; comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase ; les affections du système nerveux central comme la migraine, le vasospasme cérébral, l'hémorragie cérébrale, les oedèmes cérébraux, la dépression, l'anxiété, le stress, les troubles émotionnels, le trouble obsessionnel-compulsif, les attaques de panique, les états psychotiques, les troubles de la mémoire par exemple ; les affections du système rénal comme le vasospasme rénal, la nécrose du cortex rénal, le diabète insipide néphrogénique ; les affections du système gastrique comme le vasospasme gastrique, l'hépatocirrhose, les ulcères, la pathologie des vomissements, par exemple la nausée y compris la nausée due à une chimiothérapie, le mal des transports ; la néphropathie diabétique. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules ; des encéphalopathies hyponatrémiques ; du syndrome pulmonaire, de la maladie de Menière ; du glaucome, de la cataracte ; de l'obésité ; du diabète de type II ; de l'athérosclérose ; du syndrome de Cushing ; de la résistance à l'insuline ; de l'hypertriglycéridémie ; dans les traitements post-opératoires, notamment après une chirurgie abdominale.

Les composés selon l'invention peuvent aussi être utilisés dans le traitement ou la prévention de toutes les pathologies consécutives au stress comme la fatigue et ses syndrômes, les désordres ACTH dépendants, les troubles cardiaques, la douleur, les modifications de la vidange gastrique, de l'excrétion fécale (colite, syndrôme du colon irritable, maladie de Crohn), de la sécrétion acide, l'hyperglycémie, l'immunosuppression, les processus inflammatoires (arthrite rhumatoïde et ostéoarthrite), les infections multiples, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysorurrénalien.

Les composés selon l'invention peuvent également être utilisés comme psychostimulants, provoquant l'augmentation de l'éveil, la réactivité émotionnelle face à l'environnement et facilitant l'adaptation.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale,

le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

**1.** Composé répondant à la formule (I) :

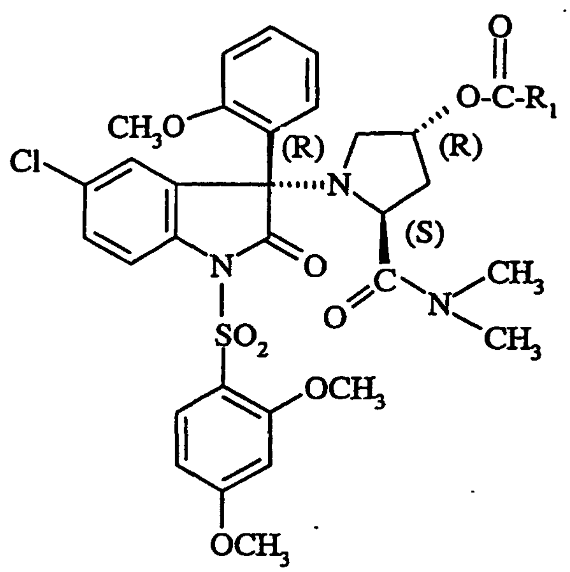

(I)

dans laquelle :

- $R_1$ représente un atome d'hydrogène, un $(C_1\text{-}C_6)$alkyle, un $(C_3\text{-}C_6)$cycloalkyle, un groupe $-CH_2CH_2COOH$ ou un groupe $-NR_2R_3$ ;

- $R_2$ et $R_3$ représentent chacun indépendamment un atome d'hydrogène ou un $(C_1-C_6)$alkyle ;

à l'état de base ou de sel d'addition à une base organique ou inorganique, ainsi qu'à l'état d'hydrate ou de solvat.

**2.** Composé selon la revendication 1 de formule (I) dans laquelle $R_1$ représente un atome d'hydrogène, un radical méthyle, un radical éthyle, un radical isopropyle, un radical cyclohexyle, un groupe $-CH_2CH_2COOH$, un groupe amino ou un groupe diméthylamino ;
à l'état de base ou de sel d'addition à une base organique ou inorganique, ainsi qu'à l'état d'hydrate ou de solvat.

**3.** Un composé choisi parmi :

- Acétate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle ;
- Propionate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino) carbonyl]-3-pyrrolidinyle ;
- Formiate de (3R,5S)-1-[(3R)-5-chloro-1-[(2,4-diméthoxyphényl)sulfonyl]-3-(2-méthoxyphényl)-2-oxo-2,3-dihydro-1*H*-indol-3-yl]-5-[(diméthylamino)carbonyl]-3-pyrrolidinyle;
- Cyclohexanecarboxylate de (3R,5S)-1-[(3R)-5-chloro-1-[(2,4-diméthoxyphényl) sulfonyl]-3-(2-méthoxyphényl)-2-oxo-2,3-dihydro-1*H*-indol-3-yl]-5-[(diméthylamino)carbonyl]-3-pyrrolidinyle;
- 2-Méthylpropanoate de (3R,5S)-1-[(3R)-5-chloro-1-[(2,4-diméthoxyphényl) sulfonyl]-3-(2-méthoxyphényl)-2-oxo-2,3-dihydro-1*H*-indol-3-yl]-5-[(diméthylamino)carbonyl]-3-pyrrolidinyle ;
- Acide 4-[[(3R,5S)-1-[(3R)-5-chloro-1-[(2,4-diméthoxyphényl)sulfonyl]-3-(2-méthoxyphényl)-2,3-dihydro-1*H*-indol-3-yl]-5-[(diméthylamino)carbonyl]-3-pyrrolidinyl]oxy]-4-oxobutanoïque ;
- (2S,4R)-4-[(aminocarbonyl)oxy]-1-[(3R)-5-chloro-1-[(2,4-diméthoxyphényl) sulfonyl]-3-(2-méthoxyphényl)-2-oxo-2,3-dihydro-1*H*-indol-3-yl]-N,N-diméthyl-2-pyrolidinecarboxamide ;
- (2S,4R)-4-[[(diméthylamino)carbonyl]oxy]-1-[(3R)-5-chloro-1-[(2,4-diméthoxyphényl)sulfonyl]-3-(2-méthoxyphényl)-2-oxo-2,3-dihydro-1*H*-indol-3-yl]-N,N-diméthyl-2-pyrrolidinecarboxamide ;

à l'état de base ou de sel d'addition à une base organique ou inorganique, ainsi qu'à l'état de d'hydrate ou de solvat.

**4.** Procédé de préparation des composés de formule (I) selon la revendication 1, **caractérisé en ce que** :

on fait réagir le (2S,4R)-1-[5-chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre, de formule :

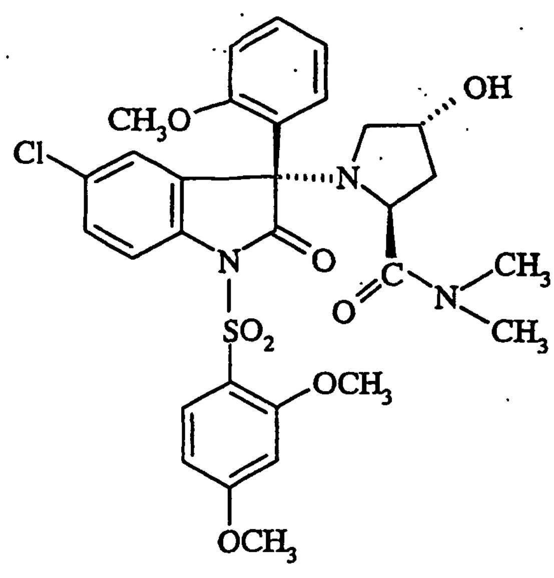

**(composé A)**

avec un dérivé fonctionnel d'un acide de formule :

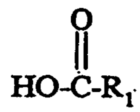

(II)

dans laquelle $R_1$ est tel que défini pour un composé de formule (I) dans la revendication 1.

**5.** Procédé de préparation des composés de formule (I) selon la revendication 1 dans laquelle $R_1$ représente un groupe $-NR_2R_3$, **caractérisé en ce que** :

a) on fait réagir le composé A tel que défini à la revendication 4, en présence d'une base, avec le chloroformiate de phényle pour obtenir le composé B de formule :

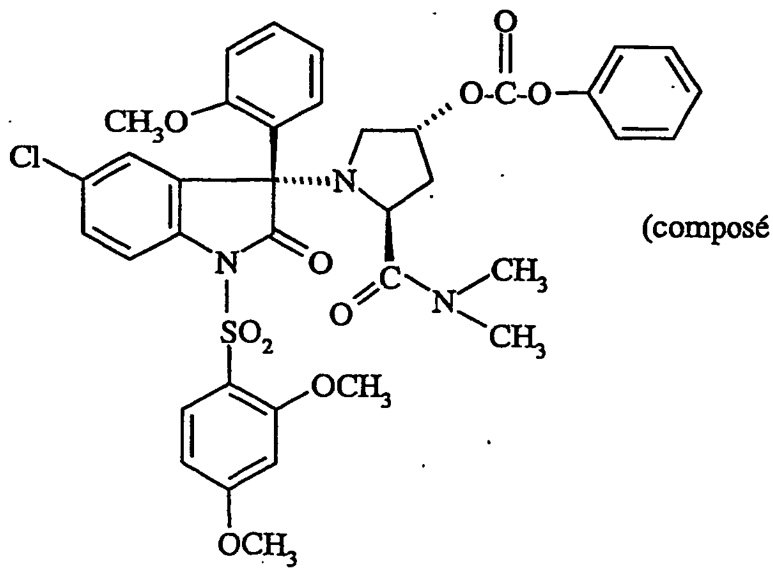

(composé B)

b) on fait réagir le composé B avec un composé de formule :

$$HNR_2R_3 \quad \text{(III)}$$

dans laquelle $R_2$ et $R_3$ sont tels que définis pour un composé de formule (I) dans la revendication 1 pour obtenir un composé de formule (I) dans laquelle $R_1 = NR_2R_3$.

**6.** Composé de formule :

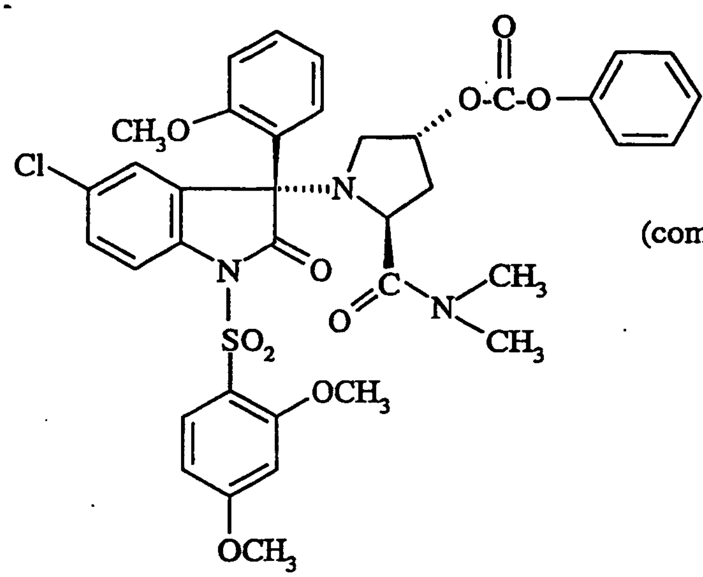

(composé B)

7. Médicament **caractérisé en ce qu'**il comprend un composé de formule (I) selon l'une quelconque des revendications 1 à 3 ou un sel d'addition de ce composé à une base pharmaceutiquement acceptable, ou encore un hydrate ou un solvat du composé de formule (I).

8. Composition pharmaceutique, **caractérisé en ce qu'**elle comprend un composé de formule (I) selon l'une quelconque des revendications 1 à 3, ou un sel pharmaceutiquement acceptable, un hydrate ou un solvat de ce composé, ainsi qu'au moins un excipient pharmaceutiquement acceptable.

9. Utilisation d'un composé de formule (I) selon l'une quelconque des revendications 1 à 3 pour la préparation de médicaments destinés à traiter les affections cardiovasculaires, le stress, l'anxiété, la dépression, le trouble obsessionnel compulsif, les attaques de panique, les affections du système rénal, les affections du système gastrique, les cancers pulmonaires à petites cellules, l'obésité, le diabète de type I et II, la résistance à l'insuline, l'hypertriglycéridémie, l'athérosclérose, le syndrome de Cushing, la dysménorrhée et le travail prématuré.

**Patentansprüche**

1. Verbindung, die der Formel (I):

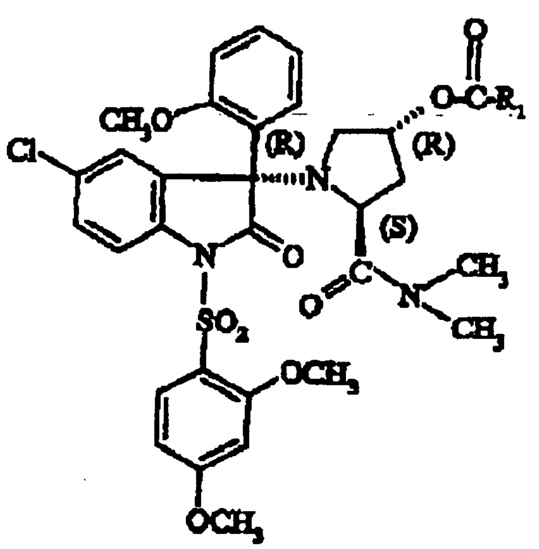

(I)

entspricht, worin:

- $R_1$ ein Wasserstoffatom, ein $(C_1-C_6)$-Alkyl, ein $(C_3-C_6)$-Cycloalkyl, eine $-CH_2CH_2COOH$-Gruppe oder eine $-NR_2R_3$-Gruppe darstellt;
- $R_2$ und $R_3$ jeweils unabhängig voneinander ein Wasserstoffatom oder ein $(C_1-C_6)$-Alkyl darstellen;

in Form einer Base oder eines Additionssalzes mit einer organischen oder anorganischen Base sowie in Form eines Hydrats oder eines Solvats.

**2.** Verbindung gemäß Anspruch 1 der Formel (I), worin $R_1$ ein Wasserstoffatom, einen Methylrest, einen Ethylrest, einen Isopropylrest, einen Cyclohexylrest, eine $-CH_2CH_2COOH$-Gruppe, eine Aminogruppe oder eine Dimethylaminogruppe darstellt;
in Form einer Base oder eines Additionssalzes mit einer organischen oder anorganischen Base sowie in Form eines Hydrats oder eines Solvats.

**3.** Eine Verbindung, die ausgewählt ist unter:

- (3R,5S)-1-[(3R)-5-Chlor-1-[(2,4-dimethoxyphenyl)sulfonyl]-3-(2-methoxyphenyl)-2-oxo-2,3-dihydro-1*H*-indol-3-yl]-5-[(dimethylamino)carbonyl]-3-pyrrolidinyl-acetat;
- (3R,5S)-1-[(3R)-5-Chlor-1-[(2,4-dimethoxyphenyl)sulfonyl]-3-(2-methoxyphenyl)-2-oxo-2,3-dihydro-1*H*-indol-3-yl]-5-[(dimethylamino)carbonyl]-3-pyrrolidinyl-propionat;
- (3R,5S)-1-[(3R)-5-Chlor-1-[(2,4-dimethoxyphenyl)sulfonyl]-3-(2-methoxyphenyl)-2-oxo-2,3-dihydro-1*H*-indol-3-yl]-5-[(dimethylamino)carbonyl]-3-pyrrolidinyl-formiat;
- (3R,5S)-1-[(3R)-5-Chlor-1-[(2,4-dimethoxyphenyl)sulfonyl]-3-(2-methoxyphenyl)-2-oxo-2,3-dihydro-1*H*-indol-3-yl]-5-[(dimethylamino)carbonyl]-3-pyrrolidinyl-cyclohexancarboxylat,
- (3R,5S)-1-[(3R)-5-Chlor-1-[(2,4-dimethoxyphenyl)sulfonyl]-3-(2-methoxyphenyl)-2-oxo-2,3-dihydro-1*H*-indol-3-yl]-5-[(dimethylamino)carbonyl]-3-pyrrolidinyl-2-methylpropanoat;
- 4-[[(3R,5S)-1-[(3R)-5-Chlor-1-[(2,4-dimethoxyphenyl)sulfonyl]-3-(2-methoxyphenyl)-2,3-dihydro-1*H*-indol-3-yl]-5-[(dimethylamino)carbonyl]-3-pyrrolidinyl]oxy]-4-oxobutansäure;
- (2S,4R)-4-[(Aminocarbonyl)oxy]-1-[(3R)-5-chlor-1-[(2,4-dimethoxyphenyl)sulfonyl]-3-(2-methoxyphenyl)-2-oxo-2,3-dihydro-1*H*-indol-3-yl]-N,N-dimethyl-2-pyrrolidincarboxamid;
- (2S,4R)-4-[[(Dimethylamino)carbonyl]oxy]-1-[(3R)-5-chlor-1-[(2,4-dimethoxyphenyl)sulfonyl]-3-(2-methoxyphenyl)-2-oxo-2,3-dihydro-1*H*-indol-3-yl]-N,N-dimethyl-2-pyrrolidincarboxamid;

in Form einer Base oder eines Additionssalzes mit einer organischen oder anorganischen Base sowie in Form eines Hydrats oder eines Solvats.

**4.** Verfahren zur Herstellung der Verbindungen der Formel (I) gemäß Anspruch 1, **dadurch gekennzeichnet, dass**:

man (2S,4R)-1-[5-Chlor-1-[(2,4-dimethoxyphenyl)sulfonyl]-3-(2-methoxyphenyl)-2-oxo-2,3-dihydro-1*H*-indol-3-yl]-4-hydroxy-N,N-dimethyl-2-pyrrolidincarboxamid, linksdrehendes Isomer, der Formel:

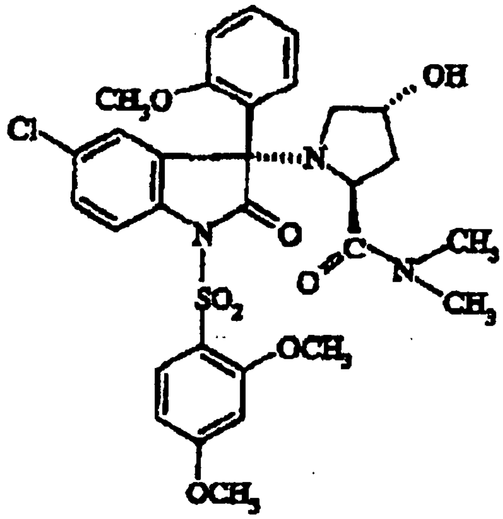

(Verbindung A)

mit einem funktionellen Derivat einer Säure der Formel:

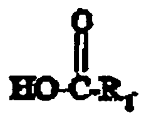

(II)

worin $R_1$ wie für eine Verbindung der Formel (I) in Anspruch 1 definiert ist, umsetzt.

**5.** Verfahren zur Herstellung der Verbindungen der Formel (I) gemäß Anspruch 1, worin $R_1$ eine $-NR_2R_3$-Gruppe darstellt, **dadurch gekennzeichnet, dass**:

a) man die Verbindung A, wie in Anspruch 4 definiert, in Gegenwart einer Base mit Phenylchlorformiat umsetzt, um die Verbindung B der Formel:

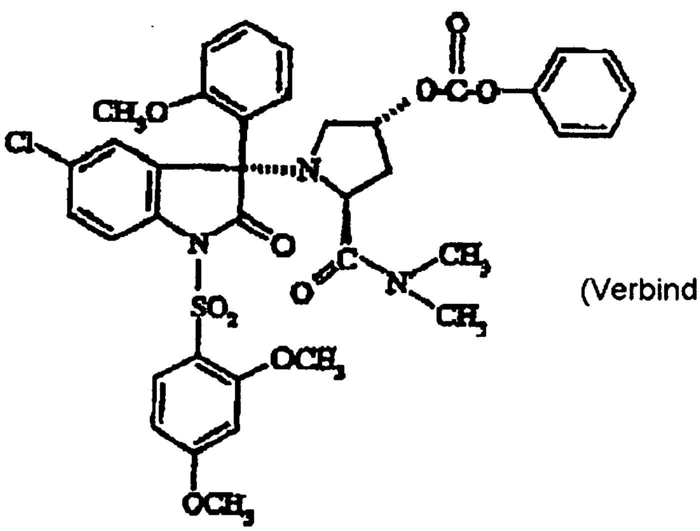

(Verbindung B)

zu erhalten,
b) man die Verbindung B mit einer Verbindung der Formel:

$$HNR_2R_3 \quad (III)$$

worin $R_2$ und $R_3$ wie für eine Verbindung der Formel (I) in Anspruch 1 definiert sind, umsetzt, um eine Verbindung der Formel (I), worin $R_1 = NR_2R_3$ zu erhalten.

**6.** Verbindung der Formel:

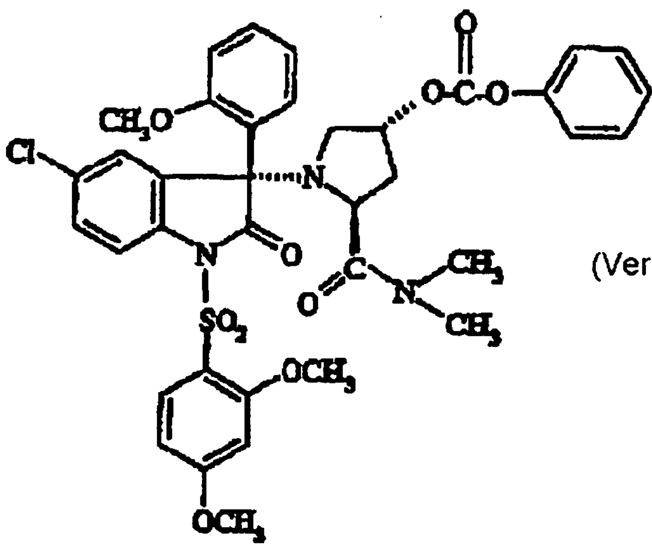

(Verbindung B)

**7.** Medikament, **dadurch gekennzeichnet, dass** es eine Verbindung der Formel (I) gemäß einem der Ansprüche 1 bis 3 oder ein Additionssalz dieser Verbindung mit einer pharmazeutisch annehmbaren Base oder auch ein Hydrat oder ein Solvat der Verbindung der Formel (I) umfasst.

**8.** Pharmazeutische Zusammensetzung, **dadurch gekennzeichnet, dass** sie eine Verbindung der Formel (I) gemäß einem der Ansprüche 1 bis 3 oder ein pharmazeutisch annehmbares Salz, ein Hydrat oder ein Solvat dieser Verbindung sowie wenigstens einen pharmazeutisch annehmbaren Hilfsstoff umfasst.

**9.** Verwendung einer Verbindung der Formel (I) gemäß einem der Ansprüche 1 bis 3 für die Herstellung von Medikamenten, die für die Behandlung von kardiovaskulären Erkrankungen, Stress, Angst, Depression, Zwangsneurosen, Panikattacken, Erkrankungen des Nierensystems, Erkrankungen des Magensystems, kleinzellige Lungenkrebse, Fettleibigkeit, Diabetes vom Typ I und II, Insulinresistenz, Hypertriglyceridämie, Atherosklerose, Cushing-Syndrom, Dysmenorrhoe und Frühgeburt bestimmt sind.

## Claims

**1.** Compound corresponding to the formula (I):

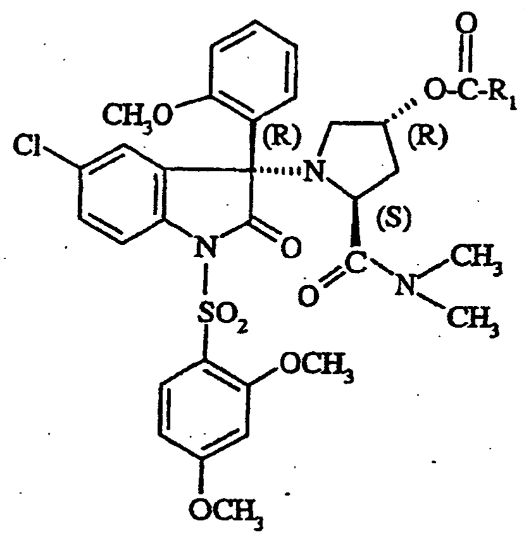

(I)

in which:

- $R_1$ represents a hydrogen atom, a $(C_1-C_6)$alkyl, a $(C_3-C_6)$ cycloalkyl, a $-CH_2CH_2COOH$ group or an $-NR_2R_3$ group;
- $R_2$ and $R_3$ each independently represent a hydrogen atom or a $(C_1-C_6)$ alkyl;

in the base form or in the form of an addition salt with an organic or inorganic base, and in the hydrate or solvate form.

**2.** Compound of formula (I) according to Claim 1, in which $R_1$ represents a hydrogen atom, a methyl radical, an ethyl radical, an isopropyl radical, a cyclohexyl radical, a $-CH_2CH_2COOH$ group, an amino group or a dimethylamino group;
in the base form or in the form of an addition salt with an organic or inorganic base, and in the hydrate or solvate form.

**3.** A compound chosen from:

- (3R,5S)-1-[(3R)-5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl acetate;
- (3R,5S)-1-[(3R)-5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl propionate;
- (3R,5S)-1-[(3R)-5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl formate;
- (3R,5S)-1-[(3R)-5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl cyclohexanecarboxylate;
- (3R,5S)-1-[(3R)-5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl 2-methylpropanoate;
- 4-[[(3R,5S)-1-[(3R)-5-Chloro-1-[(2,4-dimethoxyphenyl)-sulphonyl]-3-(2-methoxyphenyl)-2,3-dihydro-1*H*-indol-3-yl]-5-[(dimethylamino)carbonyl]-3-pyrrolidinyl]oxy]-4-oxobutanoic acid;
- (2S,4R)-4-[(Aminocarbonyl)oxy]-1-[(3R)-5-chloro-1-[(2,4-dimethoxyphenyl)sulphonyl]-3-(2-methoxyphenyl)-2-oxo-2,3-dihydro-1*H*-indol-3-yl]-N,N-dimethyl-2-pyrrolidinecarboxamide;
- (2S,4R)-4-[[(Dimethylamino)carbonyl]oxy]-1-[(3R)-5-chloro-1-[(2,4-dimethoxyphenyl)sulphonyl]-3-(2-methoxyphenyl)-2-oxo-2,3-dihydro-1*H*-indol-3-yl]-N,N-dimethyl-2-pyrrolidinecarboxamide;

in the base form or in the form of an addition salt with an organic or inorganic base, and in the hydrate or solvate form.

**4.** Process for the preparation of the compounds of formula (I) according to Claim 1, **characterized in that**:

(2S,4R)-1-[5-Chloro-1-[(2,4-dimethoxyphenyl)sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer, of formula:

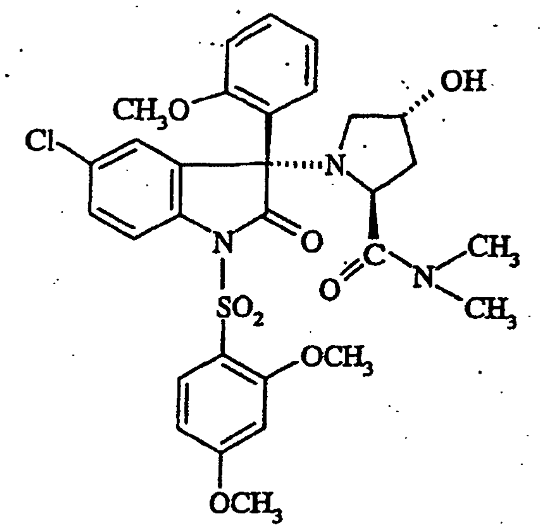

(compound A)

is reacted with a functional derivative of an acid of formula:

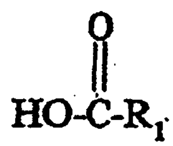

(II)

in which $R_1$ is as defined for a compound of formula (I) in Claim 1.

**5.** Process for the preparation of the compounds of formula (I) according to Claim 1 in which $R_1$ represents an $-NR_2R_3$ group, **characterized in that**:

a) compound A, as defined in Claim 4, is reacted, in the presence of a base, with phenyl chloroformate to produce compound B of formula:

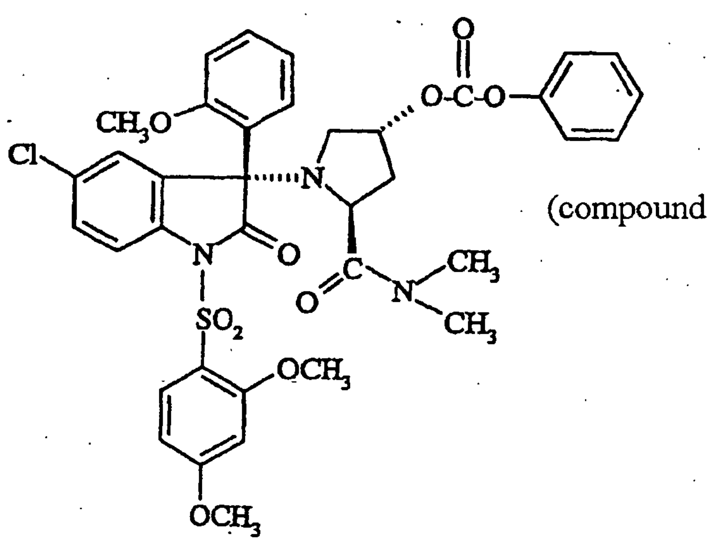

(compound B)

b) compound B is reacted with a compound of formula:

$$HNR_2R_3 \quad (III)$$

in which $R_2$ and $R_3$ are as defined for a compound of formula (I) in Claim 1, to produce a compound of formula (I) in which $R_1 = NR_2R_3$.

**6.** Compound of formula:

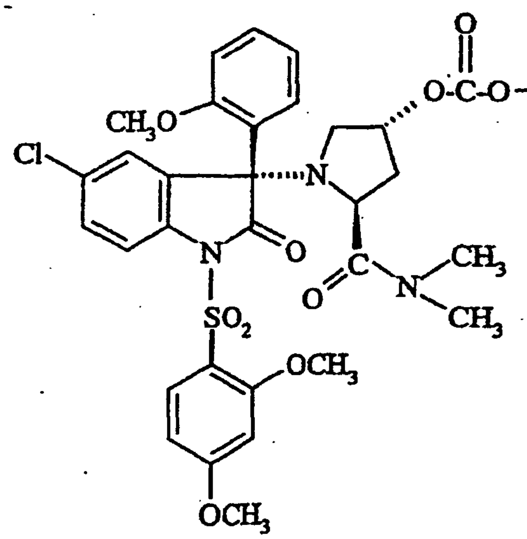

(compound B)

**7.** Medicament, **characterized in that** it comprises a compound of formula (I) according to any one of Claims 1 to 3 or an addition salt of this compound with a pharmaceutically acceptable base or a hydrate or a solvate of the compound of formula (I).

**8.** Pharmaceutical composition, **characterized in that** it comprises a compound of formula (I) according to any one of Claims 1 to 3 or a pharmaceutically acceptable salt, a hydrate or a solvate of this compound, and at least one pharmaceutically acceptable excipient.

**9.** Use of a compound of formula (I) according to any one of Claims 1 to 3 in the preparation of medicaments intended to treat cardiovascular conditions, stress, anxiety, depression, obsessive-compulsive disorder, panic attacks, conditions of the renal system, conditions of the gastric system, small cell lung cancer, obesity, type I and II diabetes, insulin resistance, hypertriglyceridaemia, atherosclerosis, Cushing's syndrome, dysmenorrhoea and premature labour.